# EUROPEAN PATENT APPLICATION

(11) **EP 3 896 173 A1**
(43) Date of publication of application: **20.10.2021**
(21) Application number: 20382308.3
(22) Date of filing: 17.04.2020
(51) Int. Cl.: C12Q 1/6883

(54) **IN VITRO METHOD FOR PREDICTING MORTALITY RISK IN PATIENTS SUFFERING FROM INFECTION WITH ORGAN FAILURE**

(71) Applicant: Fundación Instituto de Estudios Ciencias de la Salud de Castilla y León (IECSCYL-IBSAL), 37007 Salamanca (ES); Gerencia Regional de Salud de Castilla y León, 47007 Valladolid (ES); Universidad De Valladolid, 47002 Valladolid (ES)
(72) Inventor: BERMEJO MARTÍN, Jesús Francisco, 37007 Salamanca (ES); ALMANSA MORA, Raquel, 37007 Salamanca (ES); MARTÍN FERNÁNDEZ, Marta, 37007 Salamanca (ES); VAQUERO RONCERO, Luis Mario, 37007 Salamanca (ES); ESTEBAN VELASCO, María del Carmen, 37007 Salamanca (ES); TAMAYO GÓMEZ, Eduardo, 47003 Valladolid (ES); ALDECOA ÁLVAREZ-SANTULLANO, César, 37007 Salamanca (ES)
(74) Representative: Hoffmann Eitle

(57) **Abstract**

*In vitro* method for predicting mortality risk in patients suffering from infection with organ failure. The present invention refers to an *in vitro* method for predicting mortality risk, or for selecting patients with a higher mortality risk, among patients suffering from infection with organ failure, sepsis or septic shock.

## Description

### FIELD OF THE INVENTION

The present invention refers to the medical field. Particularly, the present invention refers to an *in vitro* method for predicting mortality risk, or for selecting patients with a higher mortality risk, among patients suffering from infection with organ failure, sepsis or septic shock.

### PRIOR ART

Sepsis is defined as organ dysfunction caused by a dysregulated host response to infection. Nowadays, this condition is one of the main healthcare problems in the intensive care unit (ICU), reaching to 300-480 cases per 100.000 people of incidence and leading the cause of mortality (20-30%) in the intensive care unit of the developed countries. Compared to other diseases, sepsis incidence remains higher than cerebrovascular accident (76-119 per 100.000), acute myocardial infarction (8%) and other pathologies with important social impact, such as breast cancer (25.9-94.2 per 100.000) or AIDS (24 per 100.000). Moreover, sepsis is the first cause of mortality in non-coronary ICUs, reaching the 30-50% of mortality in severe sepsis and 50-60% in septic shock.

It has been reported that the death increased by 20% in sepsis patients for each one-hour increase in door-to-antimicrobial time. Moreover, nowadays there is not an easy, quick and accurate method for predicting mortality risk in patients suffering from infection with organ failure. The gold standard used today for predicting mortality risk in sepsis patients is SOFA score (Sequential Organ Failure Assessment score). However, this score is used to track a person's status during the stay in an ICU to determine the extent of a person's organ function or rate of failure. This means that SOFA score is not designed to be used in patients during the stay in the hospital floor unit. On the other hand, SOFA is a complex method which is based on six different scores, one each for the respiratory, cardiovascular, hepatic, coagulation, renal and neurological systems.

So, in summary, nowadays, there is an unmet medical need of finding easy, quick and accurate tools for predicting mortality risk in patients suffering from infection with organ failure, sepsis or septic shock, which can be carried out not only in ICU but also in the hospital floor unit.

The present invention is focused on solving the above-mentioned problems and it is herein provided an *in vitro* method for predicting mortality risk in patients suffering from infection with organ failure, sepsis or septic shock. According to the present invention, it could be possible to get a rapid and accurate identification of patients suffering from infection with organ failure, sepsis or septic shock with a high mortality risk, also in the hospital floor unit, that would help the clinicians to provide specific treatments or measures.

### DESCRIPTION OF THE INVENTION

### Brief description of the invention

As a first step, 179 surgical patients with infection and organ failure were prospectively recruited in the three participant surgical ICUs. 22 genes encoding granule proteins sequentially expressed by granulocytes paralleling maturation were evaluated for their expression levels in blood. Area under the curve (AUC) and multivariate regression analysis were employed to evaluate the ability of gene expression to predict hospital mortality. Results were validated using the microarray data of 72 patients from a previous study.

It was observed that eleven emergency granulopoiesis genes gave rise to an AUC > 0.7 for predicting mortality risk in patients suffering from infection with organ failure, sepsis or septic shock (see **Table 1**)

### Table 1

**Table 1. Eleven emergency granulopoiesis genes with an AUC ≥ 0.7. HGNC (HUGO Gene Nomenclature Committee).**

| **Gene** | | **AUC for predicting mortality** | | | | | | |
|---|---|---|---|---|---|---|---|---|
| **Gene name** | **Gene ID** | **AUC** | **[CI 95%]** | | ***p*** | **OOP** | **Se (%)** | **Sp (%)** |
| **BPI** | HGNC: 1095 | 0.77 | 0.68 | 0.85 | < 0.001 | 598 | 72.4 | 72.7 |
| **TCN1** | HGNC: 11652 | 0.76 | 0.68 | 0.84 | < 0.001 | 358 | 79.3 | 68.0 |
| **CD24** | HGNC: 1645 | 0.75 | 0.68 | 0.82 | < 0.001 | 342 | 82.8 | 64.4 |
| **CEACAM8** | HGNC: 1820 | 0.74 | 0.66 | 0.81 | < 0.001 | 203 | 86.2 | 62.7 |
| **LCN2** | HGNC:6526 | 0.73 | 0.65 | 0.82 | < 0.001 | 3314 | 82.8 | 59.1 |
| **IL1R2** | HGNC:5994 | 0.72 | 0.63 | 0.81 | < 0.001 | 6996 | 69.0 | 73.3 |
| **LTF** | HGNC:6720 | 0.71 | 0.62 | 0.81 | < 0.001 | 1500 | 75.9 | 70.0 |
| **OLFM4** | HGNC: 17190 | 0.71 | 0.61 | 0.80 | < 0.001 | 840 | 72.4 | 68.7 |
| **STOM** | HGNC:3383 | 0.71 | 0.61 | 0.80 | < 0.001 | 2688 | 69.0 | 65.3 |
| **MMP8** | HGNC:7175 | 0.70 | 0.60 | 0.80 | 0.001 | 2240 | 79.3 | 62.0 |
| **PRTN3** | HGNC:9495 | 0.70 | 0.61 | 0.79 | 0.001 | 94.2 | 62.1 | 73.3 |

Moreover, the above cited eleven genes were combined in order to find biomarker signatures with an improved performance. For instance, **Table 2** and **Table 3** show, respectively, combinations of two or three biomarkers comprising TCN1. Most of said combinations of biomarkers are associated with an improved AUC value as compared with the AUC value of TCN1 alone. Moreover, all the AUCs included in **Table 2** and **Table 3** were compared with the AUC of SOFA by using Hanley & McNeil test and the result indicated that there are not statistically significant differences between the AUC of the combination of those genes and the AUC of SOFA (which is 0.84).

### Table 2

**Table 2. Combinations of two biomarkers comprising TCN1**

| | **AUC** | **[CI 95%]** | | ***p*** |
|---|---|---|---|---|
| **TCN1 + CEACAM8** | 0.79 | 0.71 | 0.86 | < 0.001 |
| **TCN1** + **IL1R2** | 0.78 | 0.71 | 0.86 | < 0.001 |
| **TCN1** + **MMP8** | 0.77 | 0.68 | 0.85 | < 0.001 |
| **TCN1 + PRTN3** | 0.77 | 0.69 | 0.86 | < 0.001 |
| **TCN1 +BPI** | 0.76 | 0.67 | 0.85 | < 0.001 |
| **TCN1** + **CD24** | 0.76 | 0.68 | 0.84 | < 0.001 |
| **TCN1 + LTF** | 0.75 | 0.65 | 0.85 | < 0.001 |
| **TCN1 + STOM** | 0.75 | 0.66 | 0.84 | < 0.001 |
| **TCN1+ LCN2** | 0.74 | 0.65 | 0.84 | < 0.001 |
| **TCN1 + OLFM4** | 0.74 | 0.64 | 0.84 | < 0.001 |

### Table 3

**Table 3. Combinations of three biomarkers comprising TCN1**

| | **AUC** | **[CI 95%]** | | ***p*** |
|---|---|---|---|---|
| **TCN1 + CEACAM8 + IL1R2** | 0.81 | 0.74 | 0.88 | < 0.001 |
| **TCN1 + CEACAM8 + MMP8** | 0.81 | 0.74 | 0.88 | < 0.001 |
| **TCN1 + IL1R2 + PRTN3** | 0.81 | 0.74 | 0.88 | < 0.001 |
| **TCN1** + **BPI** + **IL1R2** | 0.80 | 0.73 | 0.87 | < 0.001 |
| **TCN1** + **BPI + MMP8** | 0.79 | 0.71 | 0.87 | < 0.001 |
| **TCN1** + **BPI + CD24** | 0.79 | 0.71 | 0.86 | < 0.001 |
| **TCN1 + CD24 + CEACAM8** | 0.79 | 0.71 | 0.86 | < 0.001 |
| **TCN1 + CD24 + MMP8** | 0.79 | 0.71 | 0.86 | < 0.001 |
| **TCN1 + CD24 + PRTN3** | 0.79 | 0.72 | 0.87 | < 0.001 |
| **TCN1 + CEACAM8 + LTF** | 0.79 | 0.71 | 0.87 | < 0.001 |
| **TCN1 + CEACAM8 + OLFM4** | 0.79 | 0.71 | 0.87 | < 0.001 |
| **TCN1 + CEACAM8 + STOM** | 0.79 | 0.72 | 0.87 | < 0.001 |
| **TCN1 + CEACAM8 + PRTN3** | 0.79 | 0.71 | 0.87 | < 0.001 |
| **TCN1 + CEACAM8 + LCN2** | 0.79 | 0.71 | 0.87 | < 0.001 |
| **TCN1 + LCN2 + IL1R2** | 0.79 | 0.71 | 0.86 | < 0.001 |
| **TCN1 + IL1R2 + LTF** | 0.79 | 0.71 | 0.87 | < 0.001 |
| **TCN1 + IL1R2 + OLFM4** | 0.79 | 0.71 | 0.87 | < 0.001 |
| **TCN1 + IL1R2 + MMP8** | 0.79 | 0.72 | 0.87 | < 0.001 |
| **TCN1 + STOM + PRTN3** | 0.79 | 0.71 | 0.87 | < 0.001 |
| **TCN1 + MMP8 + PRTN3** | 0.79 | 0.72 | 0.87 | < 0.001 |
| **TCN1 + BPI + CEACAM8** | 0.78 | 0.70 | 0.86 | < 0.001 |
| **TCN1 + BPI + PRTN3** | 0.78 | 0.70 | 0.87 | < 0.001 |
| **TCN1 + CD24 + IL1R2** | 0.78 | 0.71 | 0.86 | < 0.001 |
| **TCN1 + LCN2 + PRTN3** | 0.78 | 0.70 | 0.86 | < 0.001 |
| **TCN1 + LTF + MMP8** | 0.78 | 0.69 | 0.87 | < 0.001 |
| **TCN1 + LTF + PRTN3** | 0.78 | 0.69 | 0.86 | < 0.001 |
| **TCN1 + OLFM4 + PRTN3** | 0.78 | 0.69 | 0.87 | < 0.001 |
| **TCN1 + BPI + LTF** | 0.77 | 0.68 | 0.85 | < 0.001 |
| **TCN1 + BPI + OLFM4** | 0.77 | 0.68 | 0.86 | < 0.001 |
| **TCN1 + BPI + STOM** | 0.77 | 0.69 | 0.86 | < 0.001 |
| **TCN1 + BPI + LCN2** | 0.77 | 0.68 | 0.85 | < 0.001 |
| **TCN1 + CD24 + LTF** | 0.77 | 0.69 | 0.86 | < 0.001 |
| **TCN1 + CD24 + OLFM4** | 0.77 | 0.69 | 0.86 | < 0.001 |
| **TCN1 + CD24 + LCN2** | 0.77 | 0.69 | 0.85 | < 0.001 |
| **TCN1 + IL1R2 + STOM** | 0.77 | 0.69 | 0.85 | < 0.001 |
| **TCN1 + OLFM4 + MMP8** | 0.77 | 0.68 | 0.86 | < 0.001 |
| **TCN1 + STOM + MMP8** | 0.77 | 0.68 | 0.85 | < 0.001 |
| **TCN1 + CD24 + STOM** | 0.76 | 0.67 | 0.85 | < 0.001 |
| **TCN1 + LCN2 + STOM** | 0.76 | 0.67 | 0.85 | < 0.001 |
| **TCN1 + LCN2 + MMP8** | 0.76 | 0.68 | 0.85 | < 0.001 |
| **TCN1 + LTF + STOM** | 0.76 | 0.67 | 0.85 | < 0.001 |
| **TCN1 + OLFM4 + STOM** | 0.76 | 0.66 | 0.85 | < 0.001 |
| **TCN1 + LCN2 + LTF** | 0.75 | 0.66 | 0.85 | < 0.001 |
| **TCN1 + LCN2 + OLFM4** | 0.75 | 0.65 | 0.85 | < 0.001 |
| **TCN1 + LTF + OLFM4** | 0.75 | 0.65 | 0.85 | < 0.001 |

In a further stage, the expression levels of the eleven genes included in **Table 1** [TCN1 + BPI + CD24 + CEACAM8 + LCN2 + IL1R2 + LTF + OLFM4 + STOM + MMP8 + PRTN3] were combined in the form of an additive score (Emergency Granulopoiesis Gene Expression score, EGEX). EGEX AUC to predict mortality was similar to that showed by the SOFA score (AUC [CI95%]: EGEX = 0.82 [0.75 - 0.88]; SOFA = 0.84 [0.78 - 0.90]). Multivariate analysis evidenced that EGEX was an independent predictor of mortality. Those patients with > 8 points in the EGEX score presented a four-fold increase in their risk of mortality. EGEX score in the microarray study predicted mortality with an AUC of 0.76 [0.64 - 0.87], the same to that yielded by the SOFA score.

So, in summary, the present invention shows that there is a direct relationship between the magnitude of emergency granulopoiesis and prognosis during severe infection and demonstrates that the overexpression of the above cited genes denoting emergency granulopoiesis, summarised by a combined score (EGEX), allows to predict mortality with a similar accuracy to that of SOFA, a finding that was confirmed retrospectively by using microarray data from our previous EXPRESS study.

A major strength of the present invention is that patients with infection, sepsis and septic shock were included to cover the full spectrum of organ failure degree caused by an infection. Profiling EGEX could help to estimate the mortality risk of a surgical patient with infection in an objective and easy manner, avoiding the necessity to collect the information of the six items of the SOFA score.

Consequently, the detection of the overexpression of the above cited genes contained in the EGEX score by using ddPCR could help to early identify those infected patients deserving intensive care. EGEX score could also represent a prognostic enrichment tool to selectively recruit the most severe individuals in those trials testing drugs for improving prognosis of patients with infection or sepsis.

In conclusion, the present invention is an improved alternative to the existing methods (mainly SOFA score) used today, because the present invention can be easily implemented by measuring the level of expression of the genes described above, by using known techniques like PCR, it can be carried out not only in ICU but also in the hospital floor unit, and offers an AUC value similar to the gold standard SOFA score.

So, the first embodiment of the present invention refers to an *in vitro* method for predicting mortality risk among patients suffering from infection with organ failure, sepsis or septic shock which comprises: a) Determining in a biological sample obtained from the patient the level of expression of at least TCN1, and b) wherein a variation or deviation (preferably an overexpression) of the level of expression measured in step a) is identified, with respect to the reference level of expression determined in control survivor patients suffering from infection with organ failure, sepsis or septic shock, is an indication of mortality risk.

The second embodiment of the present invention refers to an *in vitro* method for selecting patients with a higher mortality risk among patients suffering from infection with organ failure, sepsis or septic shock (i.e. prognosis enrichment) which comprises: a) Determining in a biological sample obtained from the patient the level of expression of at least TCN1, and b) wherein a variation or deviation (preferably an overexpression) of the level of expression measured in step a) is identified, with respect to the reference level of expression determined in control survivor patients suffering from infection with organ failure, sepsis or septic shock, is an indication of mortality risk.

Such as it is indicated in the first and second embodiments of the present invention, the gene TCN1 has been selected as the main biomarker because it offers a good individual AUC value (see **Table 1**) and also it is able to improve the AUC value when it is combined with other biomarkers, such as it can be seen in **Tables 2** and **3.** In any case, although TCN1 is herein defined as a preferred biomarker, any of the biomarkers included in **Table 1**, or any combination thereof, could be used in the context of the present invention, for example the following combinations associated with an AUC ≥ 0.77 for predicting mortality risk in patients suffering from infection with organ failure, sepsis or septic shock: [TCN1 + CEACAM8], [TCN1 + IL1R2], [TCN1 + MMP8], [TCN1 + PRTN3], [TCN1 + CEACAM8 + IL1R2], [TCN1 + CEACAM8 + MMP8], [TCN1 + IL1R2 + PRTN3], [TCN1 + BPI + IL1R2], [TCN1 + BPI + MMP8], [TCN1 + BPI + CD24], [TCN1 + CD24 + CEACAM8], [TCN1 + CD24 + MMP8], [TCN1 + CD24 + PRTN3], [TCN1 + CEACAM8 + LTF], [TCN1 + CEACAM8 + OLFM4], [TCN1 + CEACAM8 + STOM], [TCN1 + CEACAM8 + PRTN3], [TCN1 + CEACAM8 + LCN2], [TCN1 + LCN2 + IL1R2], [TCN1 + IL1R2 + LTF], [TCN1 + IL1R2 + OLFM4], [TCN1 + IL1R2 + MMP8], [TCN1 + STOM + PRTN3], [TCN1 + MMP8 + PRTN3], [TCN1 + BPI + CEACAM8], [TCN1 + BPI + PRTN3], [TCN1 + CD24 + IL1R2], [TCN1 + LCN2 + PRTN3], [TCN1 + LTF + MMP8], [TCN1 + LTF + PRTN3], [TCN1 + OLFM4 + PRTN3], [TCN1 + BPI + LTF], [TCN1 + BPI + OLFM4], [TCN1 + BPI + STOM], [TCN1 + BPI + LCN2], [TCN1 + CD24 + LTF], [TCN1 + CD24 + OLFM4], [TCN1 + CD24 + LCN2], [TCN1 + IL1R2 + STOM], [TCN1 + OLFM4 + MMP8], [TCN1 + STOM + MMP8], or even the following combination of eleven biomarkers: [TCN1 + BPI + CD24 + CEACAM8 + LCN2 + IL1R2 + LTF + OLFM4 + STOM + MMP8 + PRTN3].

So, in a preferred embodiment of the invention, the step a) of the above defined methods comprises determining the level of expression of: [TCN1 + CEACAM8], [TCN1 + IL1R2], [TCN1 + MMP8], [TCN1 + PRTN3], [TCN1 + CEACAM8 + IL1R2], [TCN1 + CEACAM8 + MMP8], [TCN1 + IL1R2 + PRTN3], [TCN1 + BPI + IL1R2], [TCN1 + BPI + MMP8], [TCN1 + BPI + CD24], [TCN1 + CD24 + CEACAM8], [TCN1 + CD24 + MMP8], [TCN1 + CD24 + PRTN3], [TCN1 + CEACAM8 + LTF], [TCN1 + CEACAM8 + OLFM4], [TCN1 + CEACAM8 + STOM], [TCN1 + CEACAM8 + PRTN3], [TCN1 + CEACAM8 + LCN2], [TCN1 + LCN2 + IL1R2], [TCN1 + IL1R2 + LTF], [TCN1 + IL1R2 + OLFM4], [TCN1 + IL1R2 + MMP8], [TCN1 + STOM + PRTN3], [TCN1 + MMP8 + PRTN3], [TCN1 + BPI + CEACAM8], [TCN1 + BPI + PRTN3], [TCN1 + CD24 + IL1R2], [TCN1 + LCN2 + PRTN3], [TCN1 + LTF + MMP8], [TCN1 + LTF + PRTN3], [TCN1 + OLFM4 + PRTN3], [TCN1 + BPI + LTF], [TCN1 + BPI + OLFM4], [TCN1 + BPI + STOM], [TCN1 + BPI + LCN2], [TCN1 + CD24 + LTF], [TCN1 + CD24 + OLFM4], [TCN1 + CD24 + LCN2], [TCN1 + IL1R2 + STOM], [TCN1 + OLFM4 + MMP8], [TCN1 + STOM + MMP8], or even of the following combination of eleven biomarkers: [TCN1 + BPI + CD24 + CEACAM8 + LCN2 + IL1R2 + LTF + OLFM4 + STOM + MMP8 + PRTN3].

In a preferred embodiment of the invention, the above cited methods comprise a) Determining the level of expression of any of the combinations of biomarkers included in the present invention, in a biological sample obtained from the patient, b) processing the expression level values in order to obtain a risk score and c) wherein if a deviation or variation of the risk score value obtained for any of the above cited combinations of biomarkers is identified, as compared with a reference value, this is indicative of mortality risk.

In a preferred embodiment of the invention, the method is performed within 24 hours from the patient admission.

In a preferred embodiment of the invention, the biological sample is whole blood, serum or plasma.

In a preferred embodiment the above cited genes are overexpressed as compared with the controls.

The third embodiment of the present invention refers to the *in vitro* use of TCN1, [TCN1 + CEACAM8], [TCN1 + IL1R2], [TCN1 + MMP8], [TCN1 + PRTN3], [TCN1 + CEACAM8 + IL1R2], [TCN1 + CEACAM8 + MMP8], [TCN1 + IL1R2 + PRTN3], [TCN1 + BPI + IL1R2], [TCN1 + BPI + MMP8], [TCN1 + BPI + CD24], [TCN1 + CD24 + CEACAM8], [TCN1 + CD24 + MMP8], [TCN1 + CD24 + PRTN3], [TCN1 + CEACAM8 + LTF], [TCN1 + CEACAM8 + OLFM4], [TCN1 + CEACAM8 + STOM], [TCN1 + CEACAM8 + PRTN3], [TCN1 + CEACAM8 + LCN2], [TCN1 + LCN2 + IL1R2], [TCN1 + IL1R2 + LTF], [TCN1 + IL1R2 + OLFM4], [TCN1 + IL1R2 + MMP8], [TCN1 + STOM + PRTN3], [TCN1 + MMP8 + PRTN3], [TCN1 + BPI + CEACAM8], [TCN1 + BPI + PRTN3], [TCN1 + CD24 + IL1R2], [TCN1 + LCN2 + PRTN3], [TCN1 + LTF + MMP8], [TCN1 + LTF + PRTN3], [TCN1 + OLFM4 + PRTN3], [TCN1 + BPI + LTF], [TCN1 + BPI + OLFM4], [TCN1 + BPI + STOM], [TCN1 + BPI + LCN2], [TCN1 + CD24 + LTF], [TCN1 + CD24 + OLFM4], [TCN1 + CD24 + LCN2], [TCN1 + IL1R2 + STOM], [TCN1 + OLFM4 + MMP8], [TCN1 + STOM + MMP8], or even of the following combination of eleven biomarkers: [TCN1 + BPI + CD24 + CEACAM8 + LCN2 + IL1R2 + LTF + OLFM4 + STOM + MMP8 + PRTN3] for predicting mortality risk among patients suffering from infection with organ failure, sepsis or septic shock or for selecting patients with a higher mortality risk among patients suffering from infection with organ failure, sepsis or septic shock.

The fourth embodiment of the present invention refers to a kit adapted for predicting mortality risk among patients suffering from infection with organ failure, sepsis or septic shock or for selecting patients with a higher mortality risk among patients suffering from infection with organ failure, sepsis or septic shock which comprises: a) Tools or media for obtaining a whole blood, serum or plasma sample from the patient, and b) Tools or media for measuring the expression level of TCN1, [TCN1 + CEACAM8], [TCN1 + IL1R2], [TCN1 + MMP8], [TCN1 + PRTN3], [TCN1 + CEACAM8 + IL1R2], [TCN1 + CEACAM8 + MMP8], [TCN1 + IL1R2 + PRTN3], [TCN1 + BPI + IL1R2], [TCN1 + BPI + MMP8], [TCN1 + BPI + CD24], [TCN1 + CD24 + CEACAM8], [TCN1 + CD24 + MMP8], [TCN1 + CD24 + PRTN3], [TCN1 + CEACAM8 + LTF], [TCN1 + CEACAM8 + OLFM4], [TCN1 + CEACAM8 + STOM], [TCN1 + CEACAM8 + PRTN3], [TCN1 + CEACAM8 + LCN2], [TCN1 + LCN2 + IL1R2], [TCN1 + IL1R2 + LTF], [TCN1 + IL1R2 + OLFM4], [TCN1 + IL1R2 + MMP8], [TCN1 + STOM + PRTN3], [TCN1 + MMP8 + PRTN3], [TCN1 + BPI + CEACAM8], [TCN1 + BPI + PRTN3], [TCN1 + CD24 + IL1R2], [TCN1 + LCN2 + PRTN3], [TCN1 + LTF + MMP8], [TCN1 + LTF + PRTN3], [TCN1 + OLFM4 + PRTN3], [TCN1 + BPI + LTF], [TCN1 + BPI + OLFM4], [TCN1 + BPI + STOM], [TCN1 + BPI + LCN2], [TCN1 + CD24 + LTF], [TCN1 + CD24 + OLFM4], [TCN1 + CD24 + LCN2], [TCN1 + IL1R2 + STOM], [TCN1 + OLFM4 + MMP8], [TCN1 + STOM + MMP8], or even of the following combination of eleven biomarkers: [TCN1 + BPI + CD24 + CEACAM8 + LCN2 + IL1R2 + LTF + OLFM4 + STOM + MMP8 + PRTN3].

The fifth embodiment of the present invention refers to a method for treating or for deciding the next steps or measures to be taken with a patient suffering from infection with organ failure, sepsis or septic shock and having a high mortality risk (for instance recruiting those individuals in trials testing drugs for improving prognosis of patients with infection or sepsis), which comprises a previous or initial step wherein the patient is diagnosed or identified using the above cited methods of the invention.

For the purpose of the present invention the following terms are defined:
- The expression "infection with organ failure" refers to the situation where the patient has been invaded by disease-causing agents (pathogens) causing an organ dysfunction. Precisely, "infection with organ failure" refers to the situation where there is a documented or suspected infection and 1 point is scored on SOFA scale.
- The expression "sepsis" refers to a life-threatening condition that arises when the body's response to infection causes injury to its tissues and organs. Precisely, according to the "Sepsis-3" criteria, "sepsis" refers to the situation where there is a documented or suspected infection in the presence of multiorgan failure, defined as an acute increase of 2 or more points on the SOFA scale, assuming a score of 0 in those patients where the previous existence of organ failure is not known.
- The term "septic shock" is a potentially fatal medical condition that occurs when sepsis, which is organ injury or damage in response to infection, leads to dangerously low blood pressure and abnormalities in cellular metabolism. Precisely, according to the "Sepsis-3" criteria, "septic shock" refers to the situation where there is sepsis associated with persistent hypotension that requires the use of vasopressors to maintain a MAP (Mean Arterial Pressure) > 65 mmHg and the presence of lactate > 2 mmol/L, despite performing correct fluid therapy.
- The expression "reference level of expression determined in control survivor patients suffering from infection with organ failure, sepsis or septic shock", refer to a "reference value" of the concentration level of the biomarkers. If the level of expression of the biomarkers measured in patients suffering from infection with organ failure, sepsis or septic shock is higher or lower, preferably higher, as compared with said "reference value", this is an indication of mortality risk.
- A "reference value" can be a threshold value or a cut-off value. Typically, a "threshold value" or "cut-off value" can be determined experimentally, empirically, or theoretically. A threshold value can also be arbitrarily selected based upon the existing experimental and/or clinical conditions, as would be recognized by a person of ordinary skilled in the art. The threshold value has to be determined in order to obtain the optimal sensitivity and specificity according to the function of the test and the benefit/risk balance (clinical consequences of false positive and false negative). Preferably, the person skilled in the art may compare the biomarker levels (or scores) obtained according to the method of the invention with a defined threshold value. Typically, the optimal sensitivity and specificity (and so the threshold value) can be determined using a Receiver Operating Characteristic (ROC) curve based on experimental data. For example, after determining the levels of the biomarkers in a group of reference, one can use algorithmic analysis for the statistic treatment of the measured concentrations of biomarkers in biological samples to be tested, and thus obtain a classification standard having significance for sample classification. The full name of ROC curve is receiver operator characteristic curve, which is also known as receiver operation characteristic curve. It is mainly used for clinical biochemical diagnostic tests. ROC curve is a comprehensive indicator that reflects the continuous variables of true positive rate (sensitivity) and false positive rate (1-specificity). It reveals the relationship between sensitivity and specificity with the image composition method. A series of different cut-off values (thresholds or critical values, boundary values between normal and abnormal results of diagnostic test) are set as continuous variables to calculate a series of sensitivity and specificity values. Then sensitivity is used as the vertical coordinate and specificity is used as the horizontal coordinate to draw a curve. The higher the area under the curve (AUC), the higher the accuracy of diagnosis. On the ROC curve, the point closest to the far upper left of the coordinate diagram is a critical point having both high sensitivity and high specificity values. The AUC value of the ROC curve is between 1.0 and 0.5. When AUC>0.5, the diagnostic result gets better and better as AUC approaches 1. When AUC is between 0.5 and 0.7, the accuracy is low. When AUC is between 0.7 and 0.9, the accuracy is good. When AUC is higher than 0.9, the accuracy is quite high. This algorithmic method is preferably done with a computer. Existing software or systems in the art may be used for the drawing of the ROC curve, such as: MedCalc 9.2.0.1 medical statistical software, SPSS 19.0.
- The term "mortality risk" refers to the estimation of the likelihood of in-hospital death for a patient. According to the present invention, mortality risk exists when the expression levels of the biomarkers are higher or lower (i.e. there is a variation or deviation), preferably higher, as compared with said "reference value".
- By "comprising" is meant including, but it is not limited to, whatever follows the word "comprising". Thus, use of the term "comprising" indicates that the listed elements are required or mandatory, but that other elements are optional and may or may not be present.
- By "consisting of" is meant "including, and limited to", whatever follows the phrase "consisting of'. Thus, the phrase "consisting of" indicates that the listed elements are required or mandatory, and that no other elements may be present.

### Description of the figures

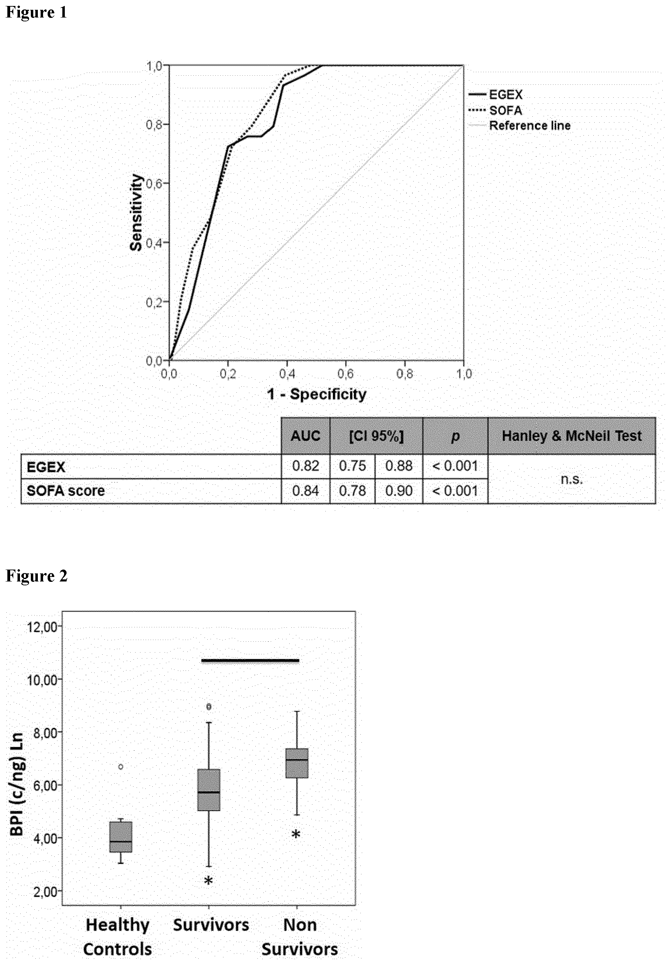

**Figure 1****.** AUC analysis for evaluating the accuracy of the EGEX score [TCN1 + BPI + CD24 + CEACAM8 + LCN2 + IL1R2 + LTF + OLFM4 + STOM + MMP8 + PRTN3] to predict hospital mortality in comparison with that of the SOFA score (derivation cohort). EGEX was built based on gene expression results obtained by ddPCR. Hanley and McNeil test was employed to identify significant differences between both AUCs.

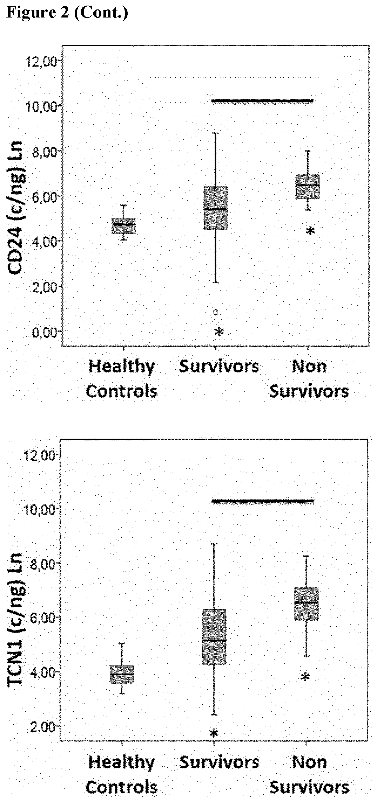

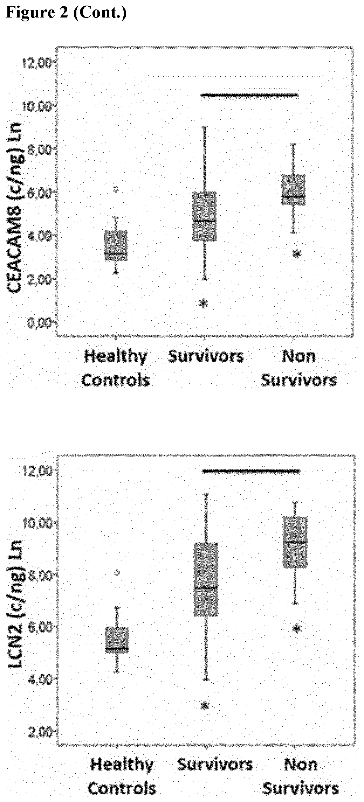

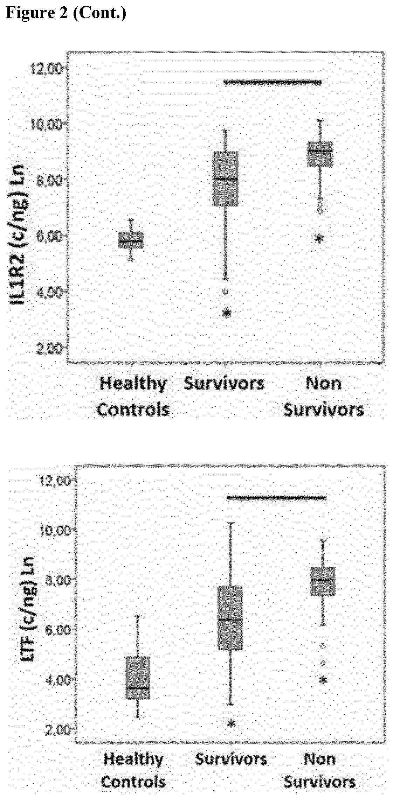

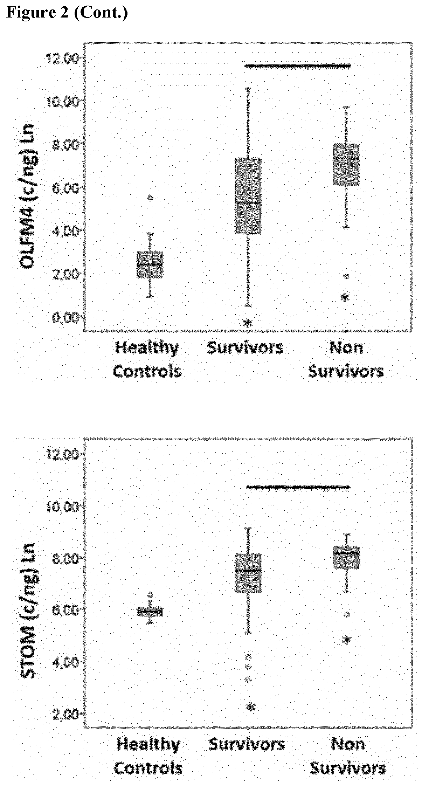

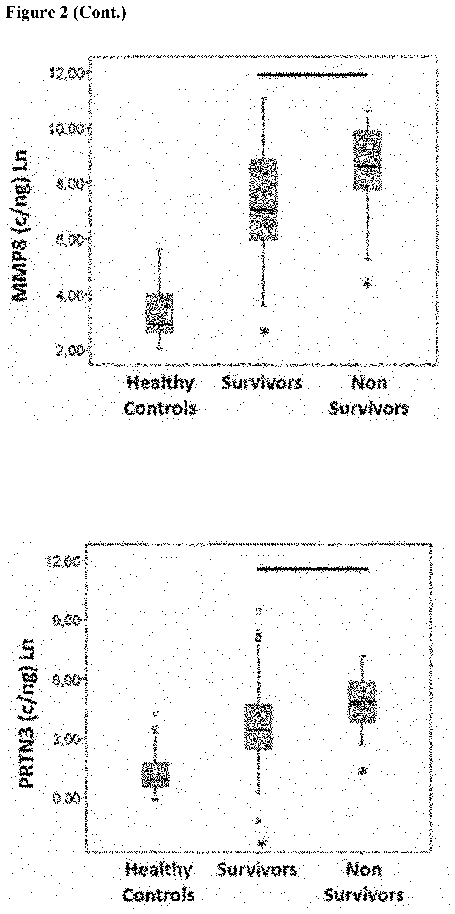

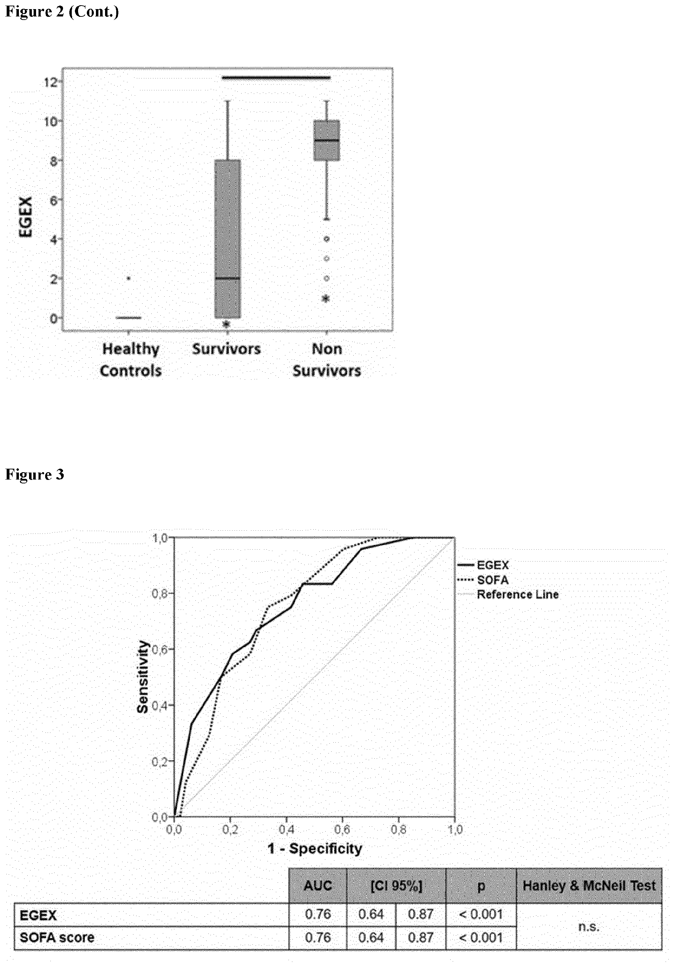

**Figure 2****.** Expression levels of the EGEX score [TCN1 + BPI + CD24 + CEACAM8 + LCN2 + IL1R2 + LTF + OLFM4 + STOM + MMP8 + PRTN3] and of the eleven genes composing it. c/ng: copies of cDNA per nanogram of initial mRNA analyzed; Ln: napierian logarithm.
**Figure 3****.** AUC analysis for evaluating the accuracy of the EGEX score [TCN1 + BPI + CD24 + CEACAM8 + LCN2 + IL1R2 + LTF + OLFM4 + STOM + MMP8 + PRTN3] to predict hospital mortality in comparison with that of the SOFA score (validation cohort). EGEX was built based on gene expression results obtained by microarrays. Hanley and McNeil test was employed to identify significant differences between both AUCs.

### Detailed description of the invention

### Example 1. Materials and Methods

### Example 1.1. Patients and controls

179 surgical patients with infection and organ failure were prospectively recruited in the surgical ICUs of the three participant hospitals from January 2017 to January 2019. Presence of infection was defined following the CDC/NHSN Surveillance Definitions for Specific Types of Infections. Presence of organ failure was assessed by using the Sequential Organ Failure Assessment (SOFA) score. Sepsis and septic shock were defined using the SEPSIS-3 consensus. A specific standard survey was employed in the participant hospitals to collect the clinical data, including medical history, physical examination and hematological, biochemical, radiological, microbiological investigations. 16 healthy controls of similar age and sex characteristics of the cases were recruited from the Center of Hemodonation and Hemotherapy of Castilla y León (CHEMCYL, Valladolid, Spain). A validation cohort composed by 72 patients was used to retrospectively validate the results for the EGEX for predicting mortality.

The study was approved by the respective Committees for Ethics in Clinical Research of the three participating hospitals (Hospital Clínico Universitario de Valladolid, CE-HCUV), (Hospital Universitario Rio Hortega de Valladolid, CE-HURH), and (Hospital Clínico Universitario de Salamanca, CE-CAUSA). Methods were carried out in accordance with current Spanish law for Biomedical Research (2007) and fulfill the standards indicated by the Declaration of Helsinki. Written informed consent was obtained from the patients' relatives or their legal representative before enrolment.

Standard cultures in biological samples guided by the presumptive source of the infection were performed to assess the presence of bacterial and fungal infection. Potentially contaminant microorganisms were not considered.

### Example 2. Quantification of emergency granulopoiesis gene expression levels

22 genes encoding granule proteins which are sequentially expressed in the bone marrow by granulocytes paralleling maturation were evaluated for their expression levels in blood. Genes were selected based on the results of our EXPRESS (Gene Expression in Sepsis) microarray study [Almansa R, Heredia-Rodríguez M, Gomez-Sanchez E, Andaluz-Ojeda D, Iglesias V, Rico L, Ortega A, Gomez-Pesquera E, Liu P, Aragón M, Eiros JM, Jiménez-Sousa MÁ, Resino S, Gómez-Herreras I, Bermejo-Martín JF, Tamayo E: Transcriptomic correlates of organ failure extent in sepsis. J Infect 2015; 70:445-56], which compared gene expression profiles of surgical patients with and without sepsis (microarray data from this study are available at the Array Express microarray data repository with code E-MTAB-1548). A sample of 2.5 mL of blood was collected by using PaxGene (BD) venous blood vacuum collection tubes in the first 12 hours following emergence of organ failure. Blood from healthy individuals was collected at the moment of donation. Total RNA was extracted from blood samples using the PAXgene Blood RNA System (PreAnalytix, Hombrechtikon, Switzerland). The evaluation of concentration and quality was performed by spectrometry (Nano-Drop ND1000, NanoDrop Technologies,Wilmington, DE). cDNA was generated from each sample on a Techne TC-512 thermal cycler (Bibby-Scientific, Staffordshire, OSA, UK) starting from 1000 ng of mRNA by using iScript Advanced cDNA Synthesis Kit (BioRad, cat: 1725038). The obtained volume of cDNA (20 uL) was further diluted (1/25), and 2.5 uL (5 ng of total mRNA) were employed for quantification of target gene expression using ddPCR on a BioRad QX200 platform (California, USA), according to the manufacturer instructions. Expression of the genes selected for the study was quantified using predesigned TaqMan Assay Primer/Probe Sets, (FAM or VIC labelled MGB probes, Thermo Fisher/Scientific-Life Technologies, Waltham, MA): Matrix metallopeptidase 8 (MMP8): Hs01029057_m1; Lactotransferrin (LTF): Hs00914334_m1; Proteinase 3 (PRTN3):Hs01597752_m1; lipocalin 2/ neutrophil gelatinase associated lipocalin (LCN2/NGAL): Hs01008571_m1; Olfactomedin 4 (OLFM4): Hs00197437_m1; Elastase (ELANE): Hs00236952_m1; Myeloperoxidase (MPO): Hs00924296_m1; cathepsin G (CTSG): Hs00175195_m1; azurocidin 1 (AZU1): Hs00156049_m1; defensin alpha 4 (DEFA4): Hs00157252_m1; Bactericidal / permeability-increasing protein (BPI): Hs01552756_m1; Interleukin 18 Receptor 1 (IL18R1): Hs00977691_m1; Carcinoembryonic antigen related cell adhesion molecule 8 (CEACAM8): Hs00266198_m1; Carcinoembryonic antigen related cell adhesion molecule 6 (CEACAM6): Hs03645554_m1; Small Cell Lung Carcinoma Cluster 4 Antigen (CD24): Hs02379687_s1; transcobalamin 1 (TCN1): Hs01055542_m1; Stomatin (STOM): Hs00925242_m1; Interleukin 1 Receptor Type 2 (IL1R2): Hs00174759_m1; Cluster of Differentiation molecule 177 molecule (CD177): Hs00360669_m1; chitinase 1 (CHIT1): Hs00185753_m1; matrix metallopeptidase 9 (MMP9): Hs00957562_m1; matrix metallopeptidase 25 (MMP25): Hs00360861_m1. Briefly, ddPCR was performed using ddPCR Supermix for Probes (no dUTP), and BioRad standard reagents for droplet generation and reading. End-point PCR with 40 cycles was performed by using C1000Touch Thermal Cycler (BioRad) after splitting each sample into approximately 20,000 droplets. Next, the droplet reader used at least 10,000 droplets to determine the percentage of positive droplets and calculation of copy number of cDNA per nanogram of initial mRNA.

### Example 3. Statistical analysis

For the demographic and clinical characteristics of the patients, differences between groups were assessed using the Chi-square test for categorical variables and the Mann-Whitney U test for continuous variables when appropriate. The accuracy of each gene of emergency granulopoiesis for predicting hospital mortality was evaluated by calculating the area under the curve (AUC). Those genes yielding an AUC ≥ 0.7 to predict mortality risk were selected to form an additive score (emergency granulopoiesis gene expression score: EGEX). To build EGEX, the optimal operating point (OOP) in the AUC analysis was identified. EGEX assigned one additional point for each one of the genes which expression levels were above the OOP. We employed the Hanley and McNeil test to compare the AUC obtained with EGEX to predict mortality with that obtained with the SOFA score. The ability of EGEX as independent predictor of mortality was confirmed by using a multivariate logistic regression analysis. Those potential confounding factors which yielded *p*-values < 0.1 in the univariate analyses were introduced as adjusting variables in the multivariate analysis. Finally, results from our previous EXPRESS study (Arrays Express code E-MTAB-1548) were employed to retrospectively validate the accuracy of EGEX to predict hospital mortality, using an AUC analysis and the Hanley and McNeil test to compare it against the predictive ability of the SOFA score.

### Example 2. Results.

### Example 2.1. Clinical characteristics of the patients

SOFA ranged from 1 to 14 with a mean of 6 (SD=3.6). Survivors were younger than the non-survivor's group. Patients of both groups were similar in terms of sex composition, surgery type, source of infection and microbiological findings. The profile of comorbidities was also similar between both groups, with the exception of the diabetes mellitus, which was more frequently present in non-survivors. Prevalence of septic shock was higher in those patients who died. As expected, non-survivors showed a higher degree of organ failure as assessed by the SOFA score and they stayed longer at the ICU. Coagulation alterations were more patent in non-survivors, who had prolonged INR and lower platelet counts. In turn, lymphocyte and monocyte counts were lower in patients who did not survive.

### Example 2.2. Gene expression levels across groups

The expression levels of emergency granulopoiesis genes were higher in non-survivors compared with survivors, with the exception of those corresponding to DEFA4, MMP9, MMP25. In turn, gene expression levels in both groups were higher than those showed by the healthy controls.

### Example 2.3. Gene expression levels for predicting mortality

AUC analysis selected eleven genes to form the EGEX score for prediction of hospital mortality (**Table 1**). As evidenced by a further analysis, EGEX AUC to predict mortality was similar to that showed by the SOFA score, as evidenced by the absence of differences in the Hanley and McNeil test (**Figure 1**). Expression levels of the eleven genes forming the EGEX score in healthy controls, survivors and non-survivors are showed in **Figure 2****.** Results from the multivariate analysis evidenced that EGEX was an independent factor for predicting mortality risk in patients with infection and organ failure. The OOP for EGEX in the AUC was 8 points. Those patients with > 8 points in the EGEX score presented a four-fold increase in their risk of mortality (**Table 4**).

### Table 4

**Table 4. Multivariate analysis for evaluating mortality risk. Ln: napierian logarithm; OOP: Optimal Operating Point.**

| | **Multivariate analysis** | | | | **Multivariate analysis for the OOP** | | | |
|---|---|---|---|---|---|---|---|---|
| | **OR** | **[CI 95%]** | | ***p*** | **OR** | **[CI95%]** | | ***p*** |
| **Age** | 1.06 | 0.99 | 1.13 | 0.104 | 1.05 | 0.98 | 1.11 | 0.170 |
| **Diabetes mellitus** | 2.18 | 0.68 | 6.94 | 0.190 | 2.32 | 0.72 | 7.50 | 0.159 |
| **Abdominal surgery** | 1.94 | 0.51 | 7.38 | 0.334 | 1.83 | 0.48 | 7.05 | 0.378 |
| **Vascular surgery** | 4.28 | 0.27 | 66.93 | 0.300 | 4.19 | 0.25 | 70.59 | 0.320 |
| **Respiratory source of infection** | 3.25 | 0.84 | 12.59 | 0.088 | 3.45 | 0.88 | 13.54 | 0.076 |
| **PCT (ng/mL) Ln** | 1.34 | 0.95 | 1.90 | 0.098 | 1.38 | 0.97 | 1.96 | 0.076 |
| **SOFA score** | 1.25 | 0.99 | 1.60 | 0.066 | 1.23 | 0.96 | 1.57 | 0.098 |
| **Septic Shock** | 2.31 | 0.63 | 8.42 | 0.205 | 2.66 | 0.73 | 9.70 | 0.139 |
| **EGEX** | 1.22 | 1.03 | 1.44 | 0.022 | - | - | - | - |
| **EGEX OOP (>8)** | - | - | - | - | 4.02 | 1.27 | 12.7 | 0.018 |

### Example 2.4. Validation of the results using microarray data

Expression levels of those genes forming EGEX were employed to build the same score using the results from our previous microarray study (the EXPRESS study). Data from 72 patients with infection and organ failure were analyzed for this purpose. As evidenced by using ddPCR, microarray data showed also that the EGEX score predicted hospital mortality with similar accuracy to that of SOFA score (**Figure 3**).

## Claims

1. *In vitro* method for predicting mortality risk among patients suffering from infection with organ failure which comprises:
a. Determining in a biological sample obtained from the patient the level of expression of at least TCN1, and
b. Wherein a variation or deviation of the level of expression measured in step a) is identified, with respect to the level of expression determined in control survivor patients suffering from infection with organ failure, is an indication of mortality risk.

2. *In vitro* method for selecting patients with a higher mortality risk among patients suffering from infection with organ failure which comprises:
a. Determining in a biological sample obtained from the patient the level of expression of at least TCN1, and
b. Wherein a variation or deviation of the level of expression measured in step a) is identified, with respect to the level of expression determined in control survivor patients suffering from infection with organ failure, is an indication of mortality risk.

3. *In vitro* method, according to any of the previous claims, wherein the patients are suffering from sepsis or septic shock.

4. *In vitro* method, according to any of the previous claims, wherein the step a) comprises determining the level of expression of [TCN1 + CEACAM8], [TCN1 + IL1R2], [TCN1 + MMP8] or [TCN1 + PRTN3].

5. *In vitro* method, according to any of the previous claims, wherein the step a) comprises determining the level of expression of [TCN1 + CEACAM8 + IL1R2], [TCN1 + CEACAM8 + MMP8], [TCN1 + IL1R2 + PRTN3], [TCN1 + BPI + IL1R2], [TCN1 + BPI + MMP8], [TCN1 + BPI + CD24], [TCN1 + CD24 + CEACAM8], [TCN1 + CD24 + MMP8], [TCN1 + CD24 + PRTN3], [TCN1 + CEACAM8 + LTF], [TCN1 + CEACAM8 + OLFM4], [TCN1 + CEACAM8 + STOM], [TCN1 + CEACAM8 + PRTN3], [TCN1 + CEACAM8 + LCN2], [TCN1 + LCN2 + IL1R2], [TCN1 + IL1R2 + LTF], [TCN1 + IL1R2 + OLFM4], [TCN1 + IL1R2 + MMP8], [TCN1 + STOM + PRTN3], [TCN1 + MMP8 + PRTN3], [TCN1 + BPI + CEACAM8], [TCN1 + BPI + PRTN3], [TCN1 + CD24 + IL1R2], [TCN1 + LCN2 + PRTN3], [TCN1 + LTF + MMP8], [TCN1 + LTF + PRTN3], [TCN1 + OLFM4 + PRTN3], [TCN1 + BPI + LTF], [TCN1 + BPI + OLFM4], [TCN1 + BPI + STOM], [TCN1 + BPI + LCN2], [TCN1 + CD24 + LTF], [TCN1 + CD24 + OLFM4], [TCN1 + CD24 + LCN2], [TCN1 + IL1R2 + STOM], [TCN1 + OLFM4 + MMP8] or [TCN1 + STOM + MMP8].

6. *In vitro* method, according to any of the previous claims, wherein the step a) comprises determining the level of expression of [TCN1 + BPI + CD24 + CEACAM8 + LCN2 + IL1R2 + LTF + OLFM4 + STOM + MMP8 + PRTN3].

7. *In vitro* method, according to any of the previous claims, which comprises a) Determining the level of expression of any of the combinations of biomarkers of claims 4 to 6, in a biological sample obtained from the patient, b) processing the expression level values in order to obtain a risk score and c) wherein if a deviation or variation of the risk score value obtained for any of the above cited combinations of biomarkers is identified, as compared with a reference value, this is indicative of mortality risk.

8. *In vitro* method, according to any of the previous claims, wherein the method is performed within 24 hours from the patient admission.

9. *In vitro* method, according to any of the previous claims, wherein the biological sample is whole blood, serum or plasma.

10. *In vitro* use of TCN1 for predicting mortality risk among patients suffering from infection with organ failure or for selecting patients with a higher mortality risk among patients suffering from infection with organ failure.

11. *In vitro* use of TCN1, according to claim 10, for predicting mortality risk among patients suffering from sepsis or septic shock, or for selecting patients with a higher mortality risk among patients suffering from sepsis or septic shock.

12. *In vitro* use, according to any of the claims 10 or 11, of [TCN1 + CEACAM8], [TCN1 + IL1R2], [TCN1 + MMP8], [TCN1 + PRTN3], [TCN1 + CEACAM8 + IL1R2], [TCN1 + CEACAM8 + MMP8], [TCN1 + IL1R2 + PRTN3], [TCN1 + BPI + IL1R2], [TCN1 + BPI + MMP8], [TCN1 + BPI + CD24], [TCN1 + CD24 + CEACAM8], [TCN1 + CD24 + MMP8], [TCN1 + CD24 + PRTN3], [TCN1 + CEACAM8 + LTF], [TCN1 + CEACAM8 + OLFM4], [TCN1 + CEACAM8 + STOM], [TCN1 + CEACAM8 + PRTN3], [TCN1 + CEACAM8 + LCN2], [TCN1 + LCN2 + IL1R2], [TCN1 + IL1R2 + LTF], [TCN1 + IL1R2 + OLFM4], [TCN1 + IL1R2 + MMP8], [TCN1 + STOM + PRTN3], [TCN1 + MMP8 + PRTN3], [TCN1 + BPI + CEACAM8], [TCN1 + BPI + PRTN3], [TCN1 + CD24 + IL1R2], [TCN1 + LCN2 + PRTN3], [TCN1 + LTF + MMP8], [TCN1 + LTF + PRTN3], [TCN1 + OLFM4 + PRTN3], [TCN1 + BPI + LTF], [TCN1 + BPI + OLFM4], [TCN1 + BPI + STOM], [TCN1 + BPI + LCN2], [TCN1 + CD24 + LTF], [TCN1 + CD24 + OLFM4], [TCN1 + CD24 + LCN2], [TCN1 + IL1R2 + STOM], [TCN1 + OLFM4 + MMP8], [TCN1 + STOM + MMP8], or [TCN1 + BPI + CD24 + CEACAM8 + LCN2 + IL1R2 + LTF + OLFM4 + STOM + MMP8 + PRTN3].

13. Kit adapted for predicting mortality risk among patients suffering from infection with organ failure or for selecting patients with a higher mortality risk among patients suffering from infection with organ failure which comprises:
a. Tools or media for obtaining a whole blood, serum or plasma sample from the patient, and
b. Tools or media for measuring the expression level of TCN1, [TCN1 + CEACAM8], [TCN1 + IL1R2], [TCN1 + MMP8], [TCN1 + PRTN3], [TCN1 + CEACAM8 + IL1R2], [TCN1 + CEACAM8 + MMP8], [TCN1 + IL1R2 + PRTN3], [TCN1 + BPI + IL1R2], [TCN1 + BPI + MMP8], [TCN1 + BPI + CD24], [TCN1 + CD24 + CEACAM8], [TCN1 + CD24 + MMP8], [TCN1 + CD24 + PRTN3], [TCN1 + CEACAM8 + LTF], [TCN1 + CEACAM8 + OLFM4], [TCN1 + CEACAM8 + STOM], [TCN1 + CEACAM8 + PRTN3], [TCN1 + CEACAM8 + LCN2], [TCN1 + LCN2 + IL1R2], [TCN1 + IL1R2 + LTF], [TCN1 + IL1R2 + OLFM4], [TCN1 + IL1R2 + MMP8], [TCN1 + STOM + PRTN3], [TCN1 + MMP8 + PRTN3], [TCN1 + BPI + CEACAM8], [TCN1 + BPI + PRTN3], [TCN1 + CD24 + IL1R2], [TCN1 + LCN2 + PRTN3], [TCN1 + LTF + MMP8], [TCN1 + LTF + PRTN3], [TCN1 + OLFM4 + PRTN3], [TCN1 + BPI + LTF], [TCN1 + BPI + OLFM4], [TCN1 + BPI + STOM], [TCN1 + BPI + LCN2], [TCN1 + CD24 + LTF], [TCN1 + CD24 + OLFM4], [TCN1 + CD24 + LCN2], [TCN1 + IL1R2 + STOM], [TCN1 + OLFM4 + MMP8], [TCN1 + STOM + MMP8], or [TCN1 + BPI + CD24 + CEACAM8 + LCN2 + IL1R2 + LTF + OLFM4 + STOM + MMP8 + PRTN3].

14. Kit, according to claim 13, adapted for predicting mortality risk among patients suffering from sepsis or septic shock, or for selecting patients with a higher mortality risk among patients suffering from sepsis or septic shock.
